(19) **Europäisches Patentamt**

**European Patent Office**

**Office européen des brevets**

(11) Publication number : **0 488 609 A1**

(12) # EUROPEAN PATENT APPLICATION

(21) Application number : **91310803.1**

(22) Date of filing : **25.11.91**

(51) Int. Cl.⁵ : **A61M 15/00**

(30) Priority : **27.11.90 GB 9025705**

(43) Date of publication of application :
**03.06.92 Bulletin 92/23**

(84) Designated Contracting States :
**AT BE CH DE DK ES FR GB GR IT LI LU NL SE**

(71) Applicant : **CIBA-GEIGY AG**
**Klybeckstrasse 141**
**CH-4002 Basel (CH)**

(72) Inventor : **Reed, David**
**1 The Close**
**Upton, Nr Newark, Nottingham NG23 5SS (GB)**
Inventor : **Pallender, Christopher**
**16 Ornsay Close**
**Rise Park, Nottingham NG5 5DL (GB)**

(74) Representative : **Sharman, Thomas et al**
**CIBA-GEIGY PLC. Patent Department, Central**
**Research, Hulley Road**
**Macclesfield, Cheshire SK10 2NX (GB)**

(54) **Powder inhaler.**

(57)    A powder inhaler comprising a housing defining a storage chamber for holding powder, a dispensing chamber from which powder can be drawn by inhalation, and means for transferring a measured dose of powder from the storage chamber to the dispensing chamber characterised in that the means for transferring a measured dose of powder from the storage chamber to the dispensing chamber comprises a metering member (20) having a recess (44) of predetermined volume and being reciprocally movable between a first position in which powder from the storage chamber (14) can enter the recess and a second position in which powder in the recess is transferred to the dispensing chamber and wherein the metering member is movable by means of a plunger (16) movable at right angles to the metering member and having a cam surface (72) engaging the metering member so that depression of the plunger causes the metering member to move from its first to its second position, the metering member being spring biased to return to its first position on release of the plunger.

FIG.1

EP 0 488 609 A1

This invention relates to powder inhalers, particularly for powdered medication.

The invention relates in particular to powder inhalers of the kind comprising a housing defining a storage chamber for holding powder, a dispensing chamber from which powder can be drawn by inhalation, and means for transferring a measured dose of powder comprise a member movable from a first position in which powder from the storage chamber can enter a recess in the member, and a second position in which the recess is open to the dispensing chamber. These inhalers usually rely on the powder falling from the recess into the dispensing chamber, or being drawn from the recess by the flow of air when the powder is inhaled.

It is an object of this invention to provide an improved powder inhaler.

According to the present invention there is provided a powder inhaler of the kind referred to, in which the means for transferring a measured dose of powder comprises a metering member having a recess of predetermined volume and being reciprocally movable between a first position in which powder from the storage chamber can enter the recess and a second position in which powder in the recess is transferred to the dispensing chamber and wherein the metering member is movable by means of a plunger movable at right angles to the metering member and having a cam surface engaging the metering member so that depression of the plunger causes the metering member to move from its first to its second position, the metering member being spring biased to return to its first position on release of the plunger.

The inhaler may also be provided with a scraper member which engages in the recess as the metering member moves to its second position to scoop powder from the recess into the dispensing chamber.

Preferably, the scraper member is fixed to the housing, the recess being open at one end so that as the metering member moves to its second position the scraper member enters the recess, and there is provided a keeper member which closes the open end of the recess when the metering moves to its seond position.

The keeper member may be reciprocable in a slot formed in the metering member so as to move transversely to the direction of movement of the metering member, the keeper member being moved by engagement with part of the housing as the metering member moves between its first and second positions.

The inhaler may include an agitator member which in the first position of the metering member covers the recess in the metering member and which is moveable to uncover the recess as the metering member moves from its first position towards its second position, the agitator member having at least a portion within the storage chamber so that movement of the agitator member assists the flow of powder from the storage chamber into the recess in the metering member.

Suitably, the agitator member is connected to the metering member so that it moves in a direction opposite to that of the metering member. In one form of the invention, a pinch roller mounted in the housing is positioned between the agitator member and a surface of the metering member so that movement of the metering member in one direction moves the agitator member in the opposite direction.

The metering member may be formed in two parts, one being stationary and one being reciprocally moveable between the first position and the second position and having an aperture therethrough forming the recess, the aperture being so positioned as to carry powder from the storage chamber to the dispensing chamber which is formed by a depression in the stationary part of the metering member. The movable part may also contain a second aperture which positioned above said depression in the stationary part when the movable part is in its first position.

Preferably, the metering member is movable horizontally in a lower part of the housing, a mouthpiece extends upwards alongside the mouthpiece and the plunger is movable vertically alongside the storage chamber. This provides a particularly compact form of the inhaler.

The inhaler may also be provided with an indicator to indicate the number of doses used. This may be effected by a ratchet and gear mechanism which moves round one position each time the plunger is activated. The indicator which may be a series of numbers or a bar which increases in thickness with an increasing number of doses taken can be viewed through an aperture in the side of the inhaler.

In order to prevent the powder from becoming damp caused by moisture exhaled from the patients mouth the lower part of the housing may be provided with a desiccant chamber for containing a substance such as silica gel which will absorb any moisture.

The invention will now be described, by way of example, with reference to the accompanying drawings in which:

Figure 1 is a cross-section through an inhaler in accordance with the invention.

Figure 2 is a view similar to Figure 1 but showing the inhaler following depression of the actuating plunger, and

Figure 3 is a cross-section through another inhaler in accordance with the invention.

Referring to the drawings, an inhaler for powdered medication comprises a housing 10 defining an inhalation passage 12, a storage chamber 14, an actuating plunger 16 and a metering member 20 movable in a lower part of the housing.

The inhalation passage 12 extends upwards from a partition wall 22 to a mouthpiece 11. The wall 22 has an aperture 24 through which powder can be supplied to the lower part of the inhalation passage. At one side of the aperture, the wall 22 is bevelled at 26 to form a scraper, the function of which is described below. The storage chamber 14 extends upwards alongside the inhalation passage 12, separated from it by a wall 28.

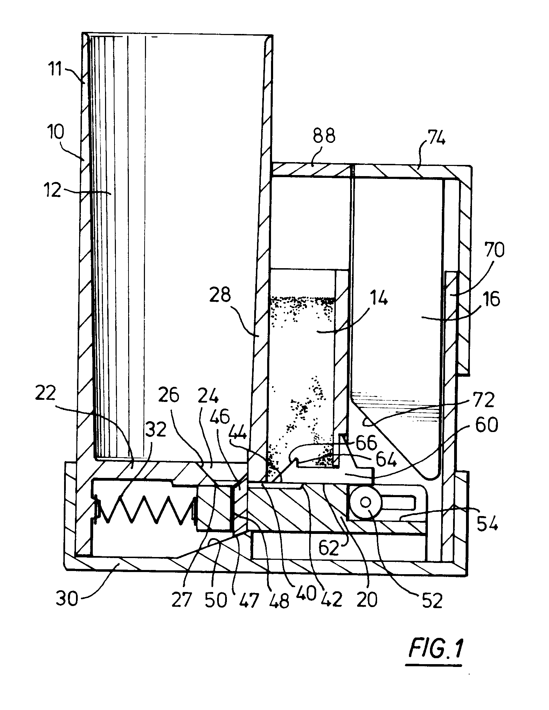

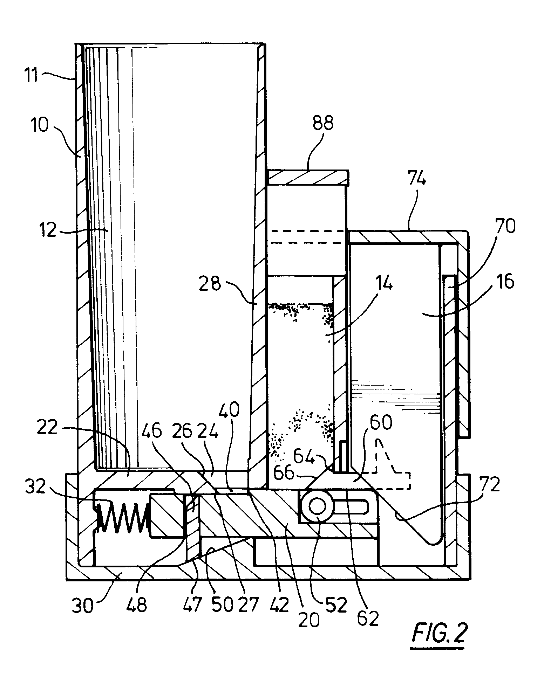

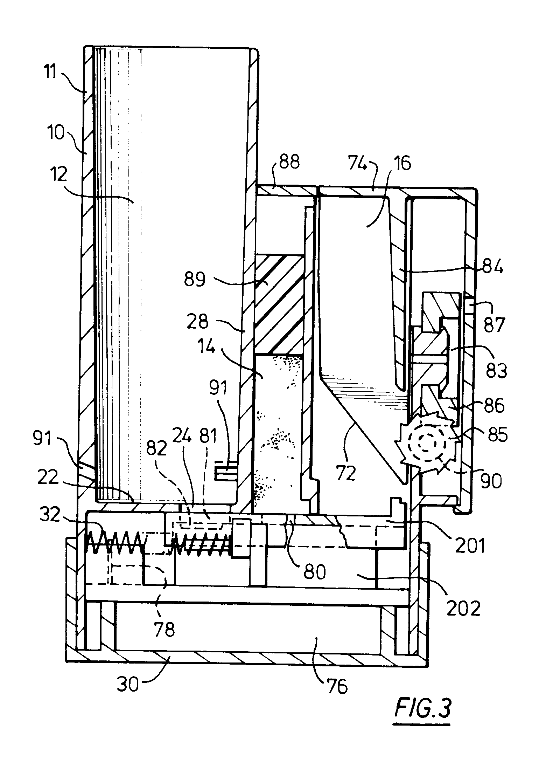

The metering member 20 is movable horizontally in a space between a lower wall 30 of the housing and the partition wall 22 and the bottom of the storage chamber 14. The metering member 20 is movable between a retracted position, shown in Figures 1 and 3 and an actuated position shown in Figure 2. A compression spring 32 biases the metering member to its retracted position. The metering member 20 in Figures 1 and 2 is formed in its upper face with a shallow recess 40, bounded at one end by a vertical wall 42 and at the other end by one face of a keeper 46. The keeper 46 slides in a vertical slot 48 in the metering member 20, between a normal position shown in Figure 1, in which it projects above the lower face 44 of the recess 40, and a retracted position, shown in Figure 2, in which it is clear of the recess. The lower face 44 of the recess 40 is at the same level as the edge 27 formed by the bevel 26 on partition wall 22, so that as the metering member 20 moves to the position shown in Figure 2, the edge 27 moves along the lower wall of the recess. The keeper 46 is moved between its upper and retracted positions by engagement of a lower bevelled face 47 of the keeper with a ramp 50 formed in the housing. A return mechanism, such as a spring, may be provided to hold the keeper in contact with the ramp.

The recess 40, defined between wall 42 and the face of keeper 46 is of a predetermined volume, corresponding to the measured dose of powder which is to be transferred from the storage chamber 14 to the inhalation passage 12.

A powder agitator 60 is slidable horizontally in the housing immediately above the metering member 20. A pinch roller 52 mounted in the housing engages the lower face 62 of the agitator 60 and also a face 54 on the metering member 20, so that as the metering member 20 moves between its retracted and actuated positions, it rotates the roller 52 to move the agitator 60 in the opposite direction.

As shown in Figure 1, when the metering member 20 is in its retracted position, the agitator 60 has a portion projecting into the storage chamber 14, and the lower face 62 of the agitator closes the recess 40 in the metering member 20. As the metering member 20 moves to its actuated position, the agitator 60 is withdrawn to uncover the recess 44. A projection 64 on the agitator is provided to agitate and loosen the powder as the agitator moves, to assist the powder to flow into the recess 44 as the member 20 moves back to its retracted position, as described below. The end 66 of the agitator is similarly bevelled to assist the flow of powder into the recess 44.

The actuating plunger 16 is slidable vertically in a guide passage formed between an outer wall 70 of the housing and the storage chamber 14. The plunger 16 is formed with an inclined lower cam face 72 which, when the plunger is depressed, engages the metering member 20 to move it from its retracted to its actuated position. On release of the plunger, it is returned to its upper position by movement of the metering member under the action of return spring 32. At the upper end of the plunger is a cap 74 which forms a button which can be pressed by the user's finger to actuate the inhaler.

In use, the storage chamber 14 is filled with powdered medication. To inhale a dose of the medication, the user presses plunger 16. As the metering member 20 moves from its retracted position to its actuated position, the keeper 46 is withdrawn, and the scraper formed by the edge 27 of wall 22 moves along the bottom of the recess 40 so that powder in the recess is scooped into the bottom of the inhalation passage 12. On release of the plunger, the metering member 20 is moved back to its retracted position by spring 32. In this position, the metering member still closes the bottom of aperture 24, whilst the keeper 46 is returned to the position shown in Figure 1, in which it engages the side wall 28 of the inhalation passage, so that powder in the inhalation passage cannot escape. The powder in the inhalation passage is inhaled through the mouthpiece 11, the powder being carried by the flow of air drawn into the inhalation passage through suitably positioned vents (not shown).

As the metering member 20 is moved back to its retracted position by the spring 32, the recess 40 moves to its position in the storage chamber 14 and powder flows into the recess to refill it. During this movement, the agitator 60 is also moved back to the position shown in Figure 1. Movement of the agitator 60 loosens powder in the chamber 14 to assist the flow of powder into the recess 40, and the agitator also acts as a scraper, pushing powder into the recess 40 and closing the recess to hold the powder in the recess when the metering member 20 and agitator 60 reach the position shown in Figure 1. During actuation of the inhaler, as the metering member 20 moves from its retracted position and the agitator 60 is withdrawn, powder in the recess 40 will again come into contact with powder in the storage chamber 14, which may help to ensure that the recess 40 is completely filled.

Referring to Figure 3, the metering member is formed of a movable part 201 and a stationary part 202. Movable part 201 has two apertures, 80, 81. Aperture 80 is a predetermined volume, corresponding to a measured dose of powder which is to be transferred from the storage chamber 14 to the inhalation passage 12. When movable part 201 is in its second position the powder is deposited in depression 82 formed in stationary part 202. Aperture 81 allows the powder in depression 82 to be inhaled through inhalation passage 12. As an alternative, movable part 201 may be short enough to leave depression 82 exposed to inhalation passage 12 when it is in its first position. In this case aperture 81 would not be needed.

Dose slide agitator 78 is provided to agitate the movable part 201 as it reaches it second position to assist the powder to deposit in depression 82 in the dispensing chamber.

Figure 3 also illustrates a counring mechanism 83. This comprises actuating finger 84, indexing gear 85 which is connected via a worm drive 90 to gear wheel 86. Each time plunger 16 is actuated, finger 84 moves indexing gear 85 round and this in turn, via the worm drive rotates gear wheel 86. Gear wheel 86 will rotate once during the life of the medicament in the inhaler. For instance if the inhaler contains 220 doses, a 10 toothed indexing gear wheel 85 and a 22 toothed gear wheel 86 would be suitable to provide for one complete rotation of gear wheel 86 after the 220 doses. A dosage indicator is provided on the wheel which is seen through aperture 87 and show how many doses have been used.

The lower part of housing 10 provides a desiccant chamber 76 for containing a desiccant material such as silica gel.

The user may inhale the powder in inhalation passage 12 either after release of the plunger, or whilst the plunger remains depressed. To assist the inhalation, air vents 91 are provided at the lower end of inhalation passage 12.

The majority of components of the inhaler, with the exception of compression spring 32, may be made of plastics material although other materials may also be used, e.g. stainless steel. The inhaler may be made of plastics material. The inhaler may be provided as a disposable unit, filled with sufficient powder for an appropriate number of doses. Alternatively, the inhaler may be refillable, for example by providing a removable cap 88 closing the top of the storage chamber, in association with a foam plug 89.

**Claims**

1. A powder inhaler comprising a housing defining a storage chamber for holding powder, a dispensing chamber from which powder can be drawn by inhalation, and means for transferring a measured dose of powder from the storage chamber to the dispensing chamber characterised in that the means for transferring a measured dose of powder from the storage chamber to the dispensing chamber comprises a metering member (20) having a recess (44) of predetermined volume and being reciprocally movable between a first position in which powder from the storage chamber (14) can enter the recess and a second position in which powder in the recess is transferred to the dispensing chamber and wherein the metering member is movable by means of a plunger (16) movable at right angles to the metering member and having a cam surface (72) engaging the metering member so that depression of the plunger causes the metering member to move from its first to its second position, the metering member being spring biased to return to its first position on release of the plunger.

2. A powder inhaler as claimed in claim 1 characterised in that a scraper member (26) is provided which engages in the recess as the metering member moves to its second position to scoop powder from the recess into the dispensing chamber.

3. A powder inhaler as claimed in claim 1 or 2 characterised in that the recess has an open end nearest the dispensing chamber and a keeper member (46) is provided which closes the open end of the recess when the metering member is in its first position and is retracted as the metering member moves to its second position.

4. A powder inhaler as claimed in claim 1 characterised in that an agitator member (60) is provided and which, in the first position of the metering member covers the recess in the metering member and which is movable to uncover the recess as the metering member moves from its first position towards its second position, the agitator member having at least a portion within the storage chamber so that movement of the agitator assists the flow of powder from the storage chamber into the recess in the metering member.

5. A powder inhaler as claimed in claim 1 characterised in that the metering member is formed in two parts one being stationary and one being reciprocally movable between the first position and the second position and having an aperture therethrough forming the recess, the aperture being so positioned as to carry powder from the storage chamber to the dispensing chamber which is formed by a depression in the stationary part of the metering member.

6. A powder inhaler as claimed in claim 5 characterised in that a dose slide agitator (78) is provided for agitate the movable part to assist the powder to deposit in the dispensing chamber.

7. A powder inhaler as claimed in claim 1 characterised in that the metering member is movable horizontally in a lower part of the housing, a mouthpiece extends upwards from the dispensing chamber, the storage chamber extends upwards alongside the mouthpiece and the plunger is movable vertically alongside the storage chamber.

8. A powder inhaler as claimed in claim 1 characterised in that the housing contains a desiccant chamber (76).

9. A powder inhaler as claimed in claim 1 characterised in that the plunger is provided with an indicator for indicating the number of doses used.

_FIG.1_

*FIG.2*

*FIG.3*

**European Patent Office**

# EUROPEAN SEARCH REPORT

Application Number

EP 91 31 0803

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| A | US-A-2 587 215 (F PRIESTLY)<br>* column 3, line 4 - column 4, line 38; figures 3,4 * | 1 | A61M15/00 |
| | | | **TECHNICAL FIELDS SEARCHED (Int. Cl.5 )** |
| | | | A61M |

The present search report has been drawn up for all claims

| Place of search | Date of completion of the search | Examiner |
|---|---|---|
| THE HAGUE | 07 FEBRUARY 1992 | VEREECKE A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.............................................................................................
& : member of the same patent family, corresponding document

EPO FORM 1503 03.82 (P0401)